# EUROPÄISCHE PATENTSCHRIFT

(11) **EP 1 290 134 B1**
(45) Veröffentlichungstag und Bekanntmachung des Hinweises auf die Patenterteilung: **06.12.2006**
(21) Anmeldenummer: 01955196.9
(22) Anmeldetag: 01.06.2001
(51) Int. Cl.: C12M 3/04, A61F 2/02, C12M 1/34

(54) **VERFAHREN ZUR BESTIMMUNG DER KONFLUENZ EINER ZELLSCHICHT AUF PORÖSEN BIOMATERIALIEN**
METHOD FOR THE DETERMINATION OF THE CONFLUENCE OF A CELL LAYER ON POROUS BIOMATERIALS
PROCEDE DE DETERMINATION DE LA CONFLUENCE D'UNE COUCHE CELLULAIRE SUR DES BIOMATERIAUX POREUX

(30) Priorität: 02.06.2000 DE 10028118
(43) Veröffentlichungstag der Anmeldung: 12.03.2003
(73) Patentinhaber: VasoTissue Technologies GmbH, 10117 Berlin (DE)
(72) Erfinder: PAULITSCHKE, Manrico, 13086 Berlin (DE); KOEPENIK, Axel, 12526 Berlin (DE)
(74) Vertreter: Wehlan, Helmut
(86) Internationale Anmeldenummer: PCT/DE2001/002109
(87) Internationale Veröffentlichungsnummer: WO 2001/091817

(56) Entgegenhaltungen:
- WO-A-93/01843
- WO-A-97/49799
- FR-A- 2 665 175
- US-A- 4 911 713

## Beschreibung

Die Erfindung betrifft ein neuartiges Verfahren zur semiquantitativen Bestimmung des Konfluenzstadiums einer Zellschicht auf der gesamten Oberfläche eines porösen Biomaterials, welches aus Kunststoffen als auch aus natürlichen Materialien gefertigt sein kann, und eine biologische Verträglichkeit gegenüber der Zellschicht aufweist. Hierbei entsteht durch Beschichtung oder Durchtränkung des porösen Biomaterials mit der Zellkultur ein Hybridkonstrukt, dessen Funktionalität entscheidend vom Grad der Geschlossenheit der Zellschicht, d.h. dem Konfluenzstadium der aufgebrachten Zellschicht, abhängt.

Verfahren zur direkten Bestimmung des Konfluenzstadiums einer Zellschicht auf Biomaterialien sind bekannt. Dabei handelt es sich um Verfahren, bei denen die Zellen radioaktiv markiert, mittels Zellfärbung und Auflichtmikroskopie visuell betrachtet oder unter Verwendung immunhistologischer Methoden untersucht werden (C. Gillis, A. Haegerstrand et al. Rapid visualization of viable and nonviable endothelium on cardiovascular prosthetic surfaces by means of fluorescent dyes. J. Thorac. Cardiovasc. Surg. 1994, 108: 1043-1048; M.B. Herring et al. 111-Indium is an unreliable in vivo label for vascular endothelial cells. Ann. Vasc. Surg. 1991, 5: 424-428).

In der US 4911713 wird ein Verfahren zur vollständigen chemischen Abdichtung einer porösen Gefäßprothese beschrieben. Die Bestimmung der Dichtigkeit erfolgt darin über die Perfusion zweier unabhängiger Kreisläufe, wobei eine Durchströmung der Wandung über eine Druckdifferenz künstlich erzeugt wird.

Die WO 97/49799 A1 und WO 93/01843 A1 beschreiben Verfahren, in denen Zellen auf porösen Materialien angezogen werden. Die Porosität der Gefäßprothese ist, wie in diesen Schriften dargestellt, offensichtlich um so größer, je weniger Zell-Zell-Kontakte zwischen den Zellen bestehen.

Oftmals sind durch die Konstruktion des Biomaterials Bedingungen gegeben, die keine ungehinderte Beurteilung der Zellschicht auf der Biomaterialoberfläche mittels optischer Methoden über die gesamte Oberfläche hinweg ermöglichen, ohne dass die Zellschicht vorher durch mechanische Veränderungen am Hybridkonstrukt freigelegt wurde. Die bekannten Anfärbetechniken zum Nachweis der Zellstrukturen auf dem Hybridkonstrukt besitzen eine zytotoxische Wirkung und führen somit zu einer irreversiblen Zellschädigung (J.D. Bancroft et al. Manual of histological techniques. Edinburgh: Churchill Livingstone, 1984; M.J.T. Visser et al. A new automated and accurate in vitro method to quantify endothelial cells attached to vascular prostheses. Thrombosis and Haemostasis, 1994, 72 (1): 146-150).

All diesen Verfahren ist als entscheidender Nachteil gemeinsam, dass mit der Freilegung und Anfärbung der Zellschicht, um diese ungehindert visuell betrachten zu können, eine funktionelle Zerstörung des Hybridkonstrukts einhergeht. Dabei kommt es zu einer irreversiblen Schädigung des Hybridkonstruktes, einerseits durch mechanische Einflüsse, um die zu untersuchende Oberfläche freizulegen, andererseits treten durch die verwendeten Agenzien am Hybridkonstrukt zytotoxische Schäden auf, die eine Weiterverwendung des untersuchten Segments für den eigentlichen Zieleinsatz ausschließen.

Um eine gesicherte Aussage über das Konfluenzstadium der Zellschicht für die gesamte Oberfläche des Hybridkonstruktes treffen zu können, muss entweder die gesamte Oberfläche oder aber, wie in den bekannten Verfahren, ein repräsentatives Teilsegment untersucht werden. Ersteres beinhaltet die bereits dargestellte irreversible Schädigung des Hybridkonstrukts, letzteres das hohe Risiko der Übertragung der Ergebnisse eines Teilsegments auf die gesamte zu betrachtende Oberfläche des Hybridkonstruktes. Daher besteht seit langem der Bedarf an einem zuverlässigen Verfahren zur Bestimmung der Zelldichte über die gesamte Hybridkonstruktoberfläche hinweg, ohne das geschaffene Konstrukt dabei in seiner Funktionalität zu beeinflussen oder gar zerstören zu müssen.

Die Aufgabe der Erfindung besteht darin, ein Verfahren darzustellen, das es ermöglicht, Aussagen über das Konfluenzstadium einer Zellschicht auf oder in einem porösen Biomaterial zu treffen und das die genannten Nachteile der bekannten Techniken überwindet.

Die Aufgabe wurde dadurch gelöst, dass das Biomaterial als durchlässige Grenzschicht in einem Zweikammersystem - Figuren (Fig.) 1 und 2 - (1, 2) über Kulturmedien-undurchlässige Adapter (3, 3') fixiert wird, wobei die vollständige Benetzung des Zellbeschichteten Biomaterials mit Kulturmedium über den gesamten Messzeitraum - gewährleistet sein muss. Die initial bestehende Porosität des als Trennschicht zwischen beiden Kammern fungierenden Biomaterials (4) ermöglicht dabei die direkte Diffusion des Kulturmediums zwischen Kammer A (1), die als statische, mit Kulturmedium gefüllte Kammer betrieben wird und über einen Druckausgleich (11) oder über Anschlussstellen (7, 7') an einen Perfusionskreislauf (5) verfügt, und einer Kammer B (2), die an einen Perfusionskreislauf (6) über die Anschlussstellen (8, 8') angeschlossen ist. Die Perfusionskreisläufe (5, 6) bestehen jeweils aus einer Pumpeinrichtung (9, 9') zur unabhängigen Steuerung beider Perfusionskreisläufe (5, 6) sowie einem Kulturmedienreservoir (10, 10'), welches auch als Druckausgleichsbehälter fungiert, die untereinander durch Schläuche verbunden sind.

Der durch die höhere Perfusion in der Kammer B (2') erzeugte höhere dynamische Druck führt zur Erniedrigung des Gesamtdrucks in Kammer B und damit zu einer in Abhängigkeit vom Grad der Porosität des Biomaterials variierenden Querströmung durch die Wandung des porösen Biomaterials (4) von Kammer A (1) in Richtung Kammer B (2). Es ist hierbei darauf zu achten, dass die beiden Kreisläufe so gesteuert werden, dass der dynamische Druck im Kreislauf 5 stets kleiner als im Kreislauf 6 bleibt, was am ehesten durch Betreiben der Kammer A (1) als statische Einheit erreicht wird. Physikalische Grundlage hierfür ist das Gesetz von Bernoulli, wonach der statische Druck in strömenden Flüssigkeiten kleiner dem in ruhenden Flüssigkeiten ist. Bei Vorliegen einer Verbindung zwischen beiden kommt es zum Einströmen von Flüssigkeit aus dem ruhenden Flüssigkeitsgebiet in das durchströmte. Das in die perfundierte Kammer B (2) übergetretene Kulturmedium wird vom Kulturmedienstrom abtransportiert und sammelt sich im externen Kulturmedienreservoir (10') des Perfusionskreislaufs (6). Dadurch erhöht sich der Volumenanteil im perfundierten Segment der Kammer nicht. Eine Bilanz über die gesamte Messkammer geht daher mit dem Verlust von Volumen an Kulturmedium und zeitgleich mit der Aufnahme von Gasmolekülen identischen Volumens in die Kammer A (1) einher. Die Ausbildung einer geschlossenen Zellschicht auf dem porösen Biomaterial (4) entspricht der Bildung einer einfachen Diffusionsbarriere hinsichtlich der Strömung zwischen beiden Kammersegmenten. Die Zellschicht übt die Funktion einer Barriere auf dem Biomaterial aus, die den perfusionsbedingten Querstrom verhindert. Mit zunehmender Zelldichte verringert sich der Volumenstrom durch die Wandung des Biomaterials, was sich in einer deutlichen Reduzierung des Volumenverlusts im nicht perfundierten Segment widerspiegelt. Mit Erreichen der Geschlossenheit der Zellschicht auf der Biomaterialoberfläche nähert sich der Volumenverlust asymptotisch einem stationären Gleichgewicht auf einem niedrigen und konstanten Niveau an.

Der Querstrom von Kulturmedium aus Kammer A (1) in Kammer B (2) kann somit, bedingt durch die unterschiedliche Dichte von in Kammer A (1) austretendem Kulturmediumvolumen und in gleichem Volumen eintretenden Gasvolumen gekennzeichnet, durch eine konstante Abnahme des Gewichts des Zweikammersystems, bestehend aus Kammer A (1) und Kammer B (2), über eine Gewichtsbestimmung der Gesamtkammer mittels einer Waage (11) messtechnisch genauestens erfasst werden. Aus der gemessenen Gewichtsreduzierung kann der übertretende Volumenanteil berechnet und in Abhängigkeit der Zeit in einer Volumenstromverlaufskurve dargestellt werden. Da diese Kurve gleichzeitig die bestehende Zahl der noch durchlässigen und somit noch nicht mit einer Zellschicht überzogenen Poren des Biomaterials reflektiert, können aus den Messwerten semiquantitative Aussagen zum Konfluenzstadium getroffen werden. Identische Aussagen zum Volumenverlust in Kammer A (1) können über eine Füllstandsanzeige (13) getroffen werden. Alternativ kann auch die Volumenzunahme im Kulturmedienreservoir (10') des Perfusionskreislaufes (6) bzw. Volumenabnahme im Kulturmedienreservoir (10) des Perfusionskreislaufes (5) durch dieselben Methoden bestimmt werden.

Die Bestimmung der Konfluenz kann ebenso dadurch erfolgen, dass sowohl Kammer A (1) als auch Kammer B (2) zur Ausbildung der Konfluenz perfundiert werden. Zur Bestimmung der Konfluenz muss dann der an Kammer A (1) angeschlossene Kreislauf (5) jedoch mit geringerem dynamischen Druck perfundiert werden, der im statischen Fall bis auf Null zurückgeht.

Das Wesen der Erfindung liegt in einem Verfahren zur semiquantitativen Bestimmung der Konfluenz einer Zellschicht auf porösen Biomaterialien, in dem mit zunehmender Konfluenz einer Zellschicht auf dem Biomaterial, das als Grenzschicht zwischen zwei perfundierbaren Kammern fixiert ist, die transversale Strömung durch die Biomaterialwandung reduziert wird, wobei der Volumenstrom messtechnisch als Indikator des Konfluenzstadiums erfasst wird. Dabei wird die transversale Strömung über eine in den beiden Kammern A und B verschiedene Strömungsgeschwindigkeiten erzeugt, die zeitlich konstant oder variierend gestaltet werden können.

Vorteilhafterweise wird das Biomaterial zur verbesserten Haftung der Zellen unter Beibehaltung der Porosität mit die Zelladhäsion verstärkenden Substanzen vorbehandelt. Die Besiedlung des Biomaterials erfolgt durch Beschichtung oder Durchtränkung mit einer oder mit verschiedenen Zellkulturen.

Die Konfluenz der Zellschicht wird bereits bei der Beschichtung oder aber durch Wachstum der Zellen unter statischen oder unter Strömungs-Bedingungen erzielt. Erfindungsgemäß wird die Bestimmung der Konfluenz durch Gewichtsbestimmung eines Kulturmedienreservoirs oder über eine Füllstandsanzeige vorgenommen.

Die Perfusionskreisläufe (5, 6) können im Gleich- oder Gegenstrom betrieben werden.

Mittels der permanenten semiquantitativen Erfassung der Messwerte kann somit überraschenderweise eine zuverlässige Aussage über das Konfluenzstadium der Zellschicht auf der gesamten Oberfläche des Biomaterials getroffen werden, ohne das Hybridkonstrukt zerstören zu müssen und ohne dabei Areale des gebildeten Hybridkonstrukts zu vernachlässigen, die nicht durch zerstörungsfreie Verfahren zur Bestimmung des Konfluenzstadiums untersucht werden können.

Die Merkmale der Erfindung gehen aus den Elementen der Ansprüchen und aus der Beschreibung hervor, wobei sowohl einzelne Merkmale als auch mehrere in Form von Kombinationen vorteilhafte Ausführungen darstellen, für die mit dieser Schrift Schutz beantragt wird.

In der genannten US 4911713 wird ein Verfahren zur vollständigen chemischen Abdichtung einer porösen Gefäßprothese beschrieben: Die Bestimmung der Dichtigkeit erfolgt über die Perfusion zweier unabhängiger Kreisläufe, wobei eine Durchströmung der Wandung über eine Druckdifferenz künstlich erzeugt wird. Dabei wird von dem Ansatz ausgegangen, dass, wenn zwischen den beiden Perfusionskreisläufen keine Druckdifferenz auftritt bzw. aufrecht zu erhalten ist, die Prothese undicht und nicht verwendbar sei (Spalte6, Zeilen 3 bis 9). Die Bestimmung der Porosität erfolgt mittels Messung einer Druckdifferenz zwischen innerem und äußerem Lumen, wobei die auftretende Druckdifferenz ausschließlich eine Aussage über den vollständigen oder den unvollständigen Verschluss erlaubt, die Druckdifferenz bzw. deren Existenz somit eine Funktion und damit den entscheidenden Parameter hinsichtlich der Porosität darstellt.

Der erfindungsgemäße Ansatz ist jedoch physikalisch völlig anderen Ursprungs: Unabhängig davon, ob die Prothese porös oder nahezu oder sogar vollständig verschlossen ist, existiert eine Druckdifferenz zwischen innerem und äußerem Kreislauf, die jedoch keineswegs eine Funktion der Porosität ist. Die Druckdifferenz in der erfindungsgemäß beschriebenen Lösung ist einzig dadurch gegeben, dass einerseits im unperfundierten Zustand der Druck ausschließlich durch die Höhe der Flüssigkeitssäule sowie die Dichte determiniert wird, der damit an jedem beliebig betrachteten Segment der Prothese im Vergleich innen/außen identische Werte aufzeigt. Andererseits entstehen durch unterschiedliche Perfusionsgeschwindigkeiten in beiden Kreisläufen zusätzlich zu diesen statischen Drücken dynamische Drücke, deren Werte proportional der Strömungsgeschwindigkeit sind und daher bei verschiedenen Perfusionsbedingungen in verschiedenen dynamischen Drücken resultieren. Die Konstanz des Gesamtdruckes als Summe aus statischem und dynamischem Druck führt dann nach Bernouilli zu einem Unterschied hinsichtlich der statischen Drücke und daraus resultierend einem transversalen Flow durch die Prothesenwandung.

Daher könnte auch eine Druckmessung, wie in der US 4911713 beschrieben wird, keinerlei Aussage hinsichtlich der Offenheit bzw. Porosität der Prothesenwandung liefern, da durch die erfindungsgemäße Lösung ein vollständiger Verschluss und eine vollständige Unterbindung einer transversalen Durchströmung der Prothesenwandung durch die Zellen nicht denkbar sind. Die Anwendung entsprechend der US-Schrift würde damit (Spalte 6, Zeile3-9) zu einem sofortigen Druckausgleich führen, unabhängig vom Grad der Konfluenz der Zellschicht. Die Prothese könnte somit quasi nie als vollständig geschlossen oder konfluent besiedelt oder teilweise besiedelt bezeichnet werden.

Die genannten WO 97/49799 A1 und WO 93/01843 A1 beschreiben Verfahren, in denen Zellen auf porösen Materialien angezogen werden: Die Porosität der Gefäßprothese ist, wie in diesen Schriften dargestellt, offensichtlich um so größer, je weniger Zell-Zell-Kontakte zwischen den Zellen bestehen. Wollte man daraus schlussfolgern, dass eine Prothese mit einer dichteren Zellschicht offensichtlich eine andere Druckdifferenz als eine Prothese mit einer weniger dichten Zellschicht und damit höherer Porosität verursacht, trifft dies sicherlich hinsichtlich der US 4911713 zu, jedoch nicht in Bezug auf die erfindungsgemäße Lösung.

Die Verfahren gemäß US 4911713, WO 97/49799 A1 und WO 93/01843 A1 verfolgen gegenüber der erfindungsgemäße Lösung einen physikalisch völlig anderen Hintergrund, und die Kombination der genannten Patentschriften führt nicht naheliegend zu dem erfindungsgemäßen Verfahren.

Die vorteilhaften Unterschiede der erfindungsgemäßen Lösung gegenüber dem Stand der Technik liegen darin , dass es mit dem anmeldungsgemäßen Verfahren erstmals möglich ist, die Abnahme der Porosität und damit die Zunahme der Zell-Zell-Kontakte über die gesamte Prothese direkt zu messen, ohne die Zell-besiedelte Prothese dem Kreislaufsystem zu entnehmen, sie in Teilen anzufärben oder anderweitig zu untersuchen und dann aus einem Teilsegment vage Schlussfolgerungen über den Gesamtzustand der Prothese zu treffen.

Die Erfindung soll anhand von Ausführungsbeispielen näher erläutert werden, ohne auf diese Beispiele beschränkt zu sein.

### Ausführungsbeispiele

### Beispiel 1

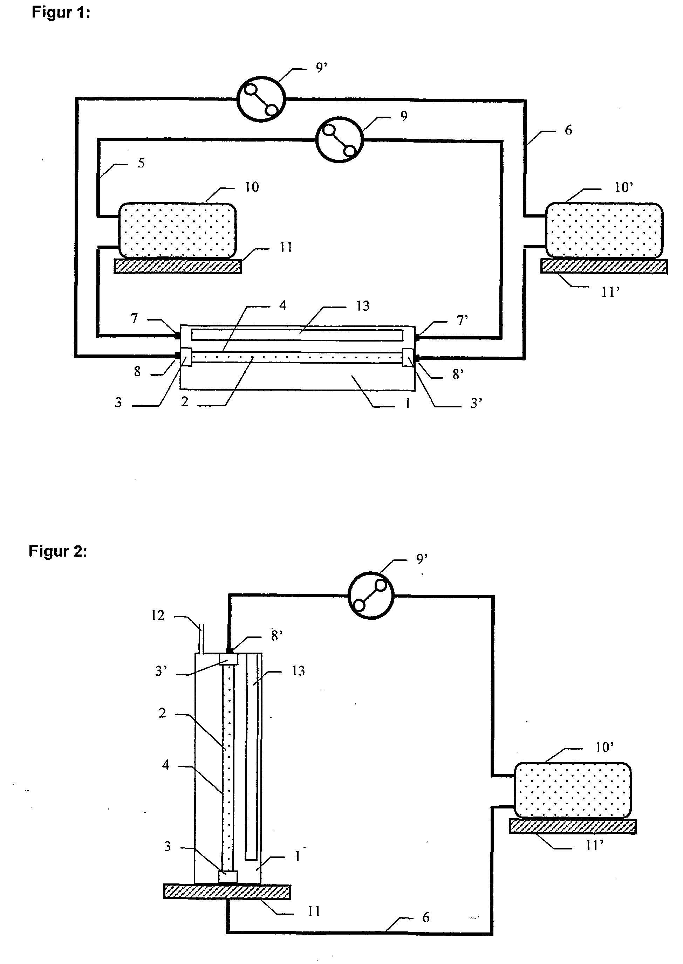

Im Zweikammersystem wird zwischen beiden Kammern (Fig.2: 1, 2) ein poröses Biomaterial (Fig.2: 4) fixiert, das durch Durchtränken mit einer Zellkultur eine initiale subkonfluente Zelldichte auf dem Biomaterial aufweist. Danach wird die Kammer B (Fig.2: 2) unter konstanter Perfusion über den Perfusionskreislauf (Fig.2: 6) betrieben, um das Konfluenzstadium der Zellen auf der Oberfläche des Hybridkonstrukts zu erreichen. Kammer A (Fig.2: 1) wird statisch betrieben und verfügt über einen Druckausgleich (Fig.2: 12). Über eine Gewichtsbestimmung (Fig.2: 11) des Zweikammersystems wird die Ausbildung der geschlossenen Zellschicht über den Volumendurchtritt durch das Biomaterial (Fig.2: 4) semiquantitativ erfasst.

### Beispiel 2

Im Zweikammersystem wird zwischen beiden Kammern (Fig.2: 1, 2) ein poröses Biomaterial (Fig.2: 4) fixiert, das durch Beschichten mit einer Zellkultur eine initiale subkonfluente Zelldichte auf dem Biomaterial aufweist. Danach wird die Kammer B (Fig.2: 2) unter sich ändernder Perfusion betrieben, um das Konfluenzstadium der Zellen auf der Oberfläche des Hybridkonstrukts zu erreichen. Kammer A (Fig.2: 1) wird statisch betrieben. Über eine Füllstandsanzeige (Fig.2: 13) in Kammer A (Fig.2: 1) oder mittels Gewichtsbestimmung (Fig.2: 11) des Zweikammersystems unter Einbeziehung der Perfusionsraten wird die Ausbildung der geschlossenen Zellschicht in Abhängigkeit vom Volumendurchtritt durch das Biomaterial semiquantitativ erfasst.

### Beispiel 3

In eine Perfusionskammer A (Fig.1: 1) wird ein poröses zur Gefäßprothese geformtes Biomaterial (Fig.1: 4) fixiert, wodurch innerhalb der Gefäßprothese die Kammer B (Fig.1: 2) gebildet wird. Das Biomaterial weist nach Beschichtung mit einer Zellkultur eine initiale subkonfluente Zelldichte auf. Danach wird das gesamte Kammersystem (Fig.1: 1, 2) über die Perfusionskreisläufe (Fig.1: 5, 6) unter sich ändernder Perfusion betrieben, um das Konfluenzstadium der Zellen auf der Oberfläche des Hybridkonstrukts zu erreichen. Die Überprüfung des Konfluenzstadiums erfolgt durch Messung des Füllstandes (Fig.1: 13) oder Gewichtsbestimmung (Fig.1: 11') des Kulturmedienreservoirs zu Kammer B (Fig.1: 10'), nachdem in Kammer A (Fig.1: 1) die Perfusion abgebrochen und diese statisch oder mit im Vergleich zur Kammer B (Fig.1: 2) geringerem dynamischen Druck weiter betrieben wird.

### Beispiel 4

In eine Perfusionskammer A (Fig.1: 1) wird ein poröses zur Gefäßprothese geformtes Biomaterial (Fig.1: 4) fixiert, wodurch innerhalb der Gefäßprothese die Kammer B (Fig.1: 2) gebildet wird. Das Biomaterial weist durch Beschichtung mit einer Zellkultur eine initiale subkonfluente Zelldichte auf dem Biomaterial auf. Die Überprüfung des Konfluenzstadiums erfolgt durch Messung des Füllstandes (Fig.1: 13) oder Gewichtsbestimmung (Fig.1: 11) des Kulturmedienreservoirs (Fig.1: 10) Kammer A (Fig.1: 1) über den Perfusionskreislauf (Fig.1: 5), der statisch oder mit im Vergleich zur Kammer B (Fig.1: 2) geringerem dynamischen Druck betrieben wird.

### Bezugszeichenindex

- 1: Kammer A
- 2: Kammer B
- 3, 3': Adapter
- 4: Biomaterial
- 5: Perfusionskreislauf Kammer A
- 6: Perfusionskreislauf Kammer B
- 7, 7': Schlauchanschlüsse Kammer A
- 8, 8': Schlauchanschlüsse Kammer B
- 9, 9': Pumpeinrichtung
- 10, 10': Kulturmedienreservoir
- 11, 11': Waage
- 12: Druckausgleich
- 13: Füllstandsanzeige

## Patentansprüche

1. Verfahren zur semiquantitativen Bestimmung der Konfluenz einer Zellschicht auf porösen Biomaterialien, **dadurch gekennzeichnet, dass** mit zunehmender Konfluenz einer Zellschicht auf dem Biomaterial, das als Grenzschicht zwischen zwei perfundierbaren Kammern fixiert ist, die transversale Strömung durch die Biomaterialwandung reduziert wird, wobei der Volumenstrom messtechnisch als Indikator des Konfluenzstadiums erfasst wird.

2. Verfahren nach Anspruch 1, **dadurch gekennzeichnet, dass** die transversale Strömung über in beiden Kammern A und B verschiedene Strömungsgeschwindigkeiten, die zeitlich konstant oder variierend gestaltet werden können, erzeugt wird.

3. Verfahren nach einem der Ansprüche 1 oder 2, **dadurch gekennzeichnet, dass** das Biomaterial zur Haftung der Zellen unter Beibehaltung der Porosität vorbehandelt wird.

4. Verfahren nach einem der Ansprüche 1 bis 3, **dadurch gekennzeichnet, dass** die Besiedlung des Biomaterials durch Beschichtung oder Durchtränkung des Biomaterials mit einer oder mit verschiedenen Zellkulturen erfolgt.

5. Verfahren nach einem der Ansprüche 1 bis 4, **dadurch gekennzeichnet, dass** die Konfluenz der Zellschicht bereits durch Beschichtung oder aber durch Wachstum der Zellen unter statischen oder Strömungs-Bedingungen erfolgt. -

6. Verfahren nach einem der Ansprüche 1 bis 5, **dadurch gekennzeichnet, dass** die Bestimmung der Konfluenz durch Gewichtsbestimmung eines Kulturmedienreservoirs erfolgt.

7. Verfahren nach einem der Ansprüche 1 bis 5, **dadurch gekennzeichnet, dass** die Bestimmung der Konfluenz über eine Füllstandsanzeige erfolgt.

8. Verfahren nach einem der Ansprüche 1 bis 7, **dadurch gekennzeichnet, dass** das Biomaterial über Kulturmedien-undurchlässige Adapter (3, 3') als durchlässige Grenzschicht in einem Zweikammersystem (1, 2) fixiert wird, wobei die vollständige Bedeckung des Zell-beschichteten Biomaterials (4) mit Kulturmedium über den gesamten Messzeitraum gewährleistet werden muss, und das Zweikammersystem aus einer Kammer A (1), die statisch betrieben wird und über einen Druckausgleich (12) verfügt, oder über Anschlussstellen (7, 7') an einen Perfusionskreislauf (5), bestehend aus einer Pumpe (9) zur unabhängigen Steuerung des Perfusionskreislaufs (5) und einem Kulturmedienreservoir (10), welches auch als Druckausgleichsbehälter fungiert, angeschlossen ist, und aus einer Kammer B, die über Anschlussstellen (8, 8') an einen Perfusionskreislauf (6), bestehend aus einer Pumpe (9') zur unabhängigen Steuerung des Perfusionskreislaufs (6) und einem Kulturmedienreservoir (10'), welches auch als Druckausgleichsbehälter fungiert, angeschlossen ist.

9. Verfahren nach Anspruch 8, **dadurch gekennzeichnet, dass** die Perfusionskreisläufe (5, 6) im Gleich- oder Gegenstrom betrieben werden können.

## Claims

1. A method for the semiquantitative determination of the confluence of a cell layer on porous biomaterials, **characterized in that** with increasing confluence of a cell layer on the biomaterial that is fixed as a boundary layer between two perfusable chambers, the transverse flow through the biomaterial wall is reduced, with the volume flow being measurement-technically detected as an indicator for the confluence state.

2. The method according to claim 1, **characterized in that** the transverse flow is generated by flow speeds that are different in the two chambers A and B and may be configured time-constant or varying.

3. The method according to either of claims 1 or 2, **characterized in that** the biomaterial is pretreated for the adhesion of the cells while maintaining the porosity.

4. The method according to any one of claims 1 through 3, **characterized in that** the colonization of the biomaterial takes place by coating or soaking of the biomaterial with one or with one or various cell culture/s.

5. The method according to any one of claims 1 through 4, **characterized in that** the confluence already takes place by coating or by the growth of the cells under static or flow conditions.

6. The method according to any one of claims 1 through 5, **characterized in that** the determination of the confluence takes place by the determination of the weight of a culture medium reservoir.

7. The method according to any one of claims 1 through 5, **characterized in that** the determination of the confluence takes place via a level indicator.

8. The method according to any one of claims 1 through 7, **characterized in that** the biomaterial is fixed as a permeable boundary layer in a two-chamber system (1, 2) by means of culture medium-impermeable adapters (3, 3'), with the complete wetting of the cell-coated biomaterial (4) with culture medium having to be guaranteed over the entire measurement period, and the two-chamber system being comprised of a chamber A (1) that is statically operated and has a pressure compensation (12), or is connected via connection points (7, 7') to a perfusion circuit (5), which is comprised of a pumping means (9) for independently controlling the perfusion circuit (5), and a culture medium reservoir (10) that acts also as a pressure compensation vessel, and of a chamber B (2) connected via connection points (8, 8') to a perfusion circuit (6), which is comprised of a pumping means (9') for independently controlling the perfusion circuit (6), and a culture medium reservoir (10') that acts also a pressure compensation vessel.

9. The method according to claim 8, **characterized in that** the perfusion circuits (5, 6) can be operated in cocurrent or countercurrent flow.

## Revendications

1. Procédé de la détermination demi-quantitative du confluent de la couche des cellules sur des biomatériaux poreux se **caractérise par le fait qu'**à cause du raccourcissement de la couche des cellules sur les biomatériaux, fixé comme le courant transversal entre deux cellules de perfusion, le courant transversal se réduit à travers la paroi des biomatériaux, et avec cela l'équipement de mesure considère le courant volumétrique comme un indicateur du stad de fusion.

2. Procédé selon la revendication 1, **caractérise en ce que** le courant transversal apparaît à cause des vitesses de courant, différentes dans les cellules A et B, qui peuvent être permanentes ou variables dans le temps.

3. Procédé selon l'une des revendications 2 ou 3, **caractérise en ce qu'**on produit le traitement préliminaire des biomatériaux pour le collage des cellules en conservant la porosité.

4. Procédé selon l'une des revendications 2 - 3, **caractérise en ce que** le remplissage des biomatériaux se produit au moyen du couvrement et de l'imbibition des biomatériaux par une ou plusieures cultures cellulaires.

5. Procédé selon l'une des revendications 1 - 4, **caractérise en ce que** la fusion de la couche cellulaire se produit par le couvrement ou par le développement des cellules dans des conditions statiques ou hydrodynamiques.

6. Procédé selon l'une des revendications 1 - 5, **caractérise en ce que** l'estimation de la fusion se réalise par l'appréciation du poids d'un réservoir à milieu nutritif.

7. Procédé selon l'une des revendications 1 - 5, **caractérise en ce que** l'estimation de la fusion se réalise par la détermination du niveau de remplissage.

8. Procédé selon l'une des revendications 1 - 7, **caractérise en ce que** les biomatériaux sont fixés par des adaptateurs impénétrables pour le milieu nutritif (3, 3') en forme de la couche frontalière dans le système à deux cellules (1, 2), en plus, le couvrement total par le milieu nutritif des biomatériaux à couche de cellules (4) doit être assuré en cours de toute la période des mesures, et le système à deux cellules est connecté à partir de la cellule A (1) qui est exploitée statiquement et possède de l'équilibrage de la pression (12) ou par l'intermédiaire des points de connection (7, 7') il est connecté au circuit de perfusion (5) qui est composé de la pompe (9), utilisé pour la commande indépendante du circuit de perfusion (5), et du réservoir à milieu nutritif, qui fonctionne ainsi que le réservoir d'accumulation ; et de la cellule B qui est connectée par l'intermédiaire des points de connection (8, 8') au circuit de perfusion (6), qui est composé de la pompe (9'), utilisé pour la commande indépendante du circuit de perfusion (6), et du réservoir à milieu nutritif (10'), qui fonctionne ainsi que le réservoir d'accumulation.

9. Procédé selon la revendication8, **caractérise en ce que** les circuits de perfusion (5,6) peuvent être exploiter avec le circuit direct et inverse.
